Europäisches Patentamt

European Patent Office

Office européen des brevets

(11) Publication number: **0 093 595**

**B1**

(12) **EUROPEAN PATENT SPECIFICATION**

(45) Date of publication of patent specification: **26.02.86**

(51) Int. Cl.⁴: **G 01 N 33/72, C 12 Q 1/28**

(21) Application number: **83302435.9**

(22) Date of filing: **29.04.83**

(54) Home diagnostic aid and method for determining the presence of occult blood.

(30) Priority: **30.04.82 US 373684**

(43) Date of publication of application:
**09.11.83 Bulletin 83/45**

(45) Publication of the grant of the patent:
**26.02.86 Bulletin 86/09**

(84) Designated Contracting States:
**BE DE FR GB NL**

(56) References cited:
**AT-B- 344 919
AT-B- 358 743
AT-B- 360 176
DE-B-2 853 300
GB-A-1 547 846
US-A-2 838 377
US-A-3 996 006
US-A-4 175 923**

(73) Proprietor: **HEMATEC CORPORATION
1221 Madison Suite 1220
Seattle Washington 98104 (US)**

(72) Inventor: **Wells, Henry J.
1145 Norwood Drive
Beaumont Texas 77706 (US)**

(74) Representative: **Jones, Michael Raymond et al
HASELTINE LAKE & CO. Hazlitt House
28 Southampton Buildings Chancery Lane
London WC2A 1AT (GB)**

Courier Press, Leamington Spa, England.

Background of the Invention

The present invention relates to a home and laboratory diagnostic aid and method for determining the presence of hemoglobin in an aqueous medium, and more particularly, to a diagnostic aid and method for determining the presence of occult blood in fecal matter present in an aqueous medium. The diagnostic aid and method of the present invention are easily used and interpreted. Most importantly, use of the diagnostic aid in accordance with the invention requires only a single manipulative step.

Over 100,000 persons in the United States are affected by cancer of the colon and rectum per year, occurring equally in both male and female. When the number of colorectal cancers occurring each year is combined with the number of cancers occurring in other digestive organs, including the esophagus and stomach, such cancers of the digestive system account for more occurrences of cancer than any other form of the disease. Contrary to many other forms of cancer, early diagnosis and treatment of digestive tract cancer does result in a cure rate of 80% to 90% of those persons affected by the disease. If, however, the disease is not detected until the later stages, the cure rate can drop drastically to 25% or less. Thus, early detection of the disease is critical to successful treatment of digestive tract cancer.

Most, but not all, cancers of the digestive tract bleed to a certain extent. This blood is deposited on and in fecal matter excreted from the digestive system. The presence of blood in fecal matter is not normally detected, however, until gross bleeding occurs, resulting in blood visible to the naked eye. Gross bleeding, however, does not normally occur until the digestive tract cancers are in advanced stages.

It is known that digestive tract cancers in the early stages also tend to bleed, giving rise to occult (hidden) blood in the fecal matter. Test equipment and test procedures have been developed for use by physicians in testing for occult blood in fecal matter. One of the most successful tests is manufactured and sold by Smith Kline Diagnostics of Sunnyvale, California, under the trademark "Hemoccult". The package for the "Hemoccult" test is disclosed in U.S. Patent No. 3,996,006 issued to J. F. Pagano. Briefly, the Pagano test employs an absorbent white paper impregnated with a guaiac reagent and encased in a special test slide having openable flaps on both sides of the test slide. To use the Pagano test slide, one must obtain a sample of fecal matter, smear it onto the guaiac-impregnated paper by opening the panel on one side of the test slide, and thereafter close the panel. A panel on the opposite side of the test slide is then opened and a developing agent, which is a stabilized solution of hydrogen peroxide and denatured alcohol, is applied to the guaiac-impregnated paper. If occult blood is present in the fecal matter smeared on the opposite side of the paper, the product of the guaiac reaction will appear as a blue substance against the white paper background, providing a positive indication of the presence of blood in the fecal matter.

Although the Pagano test is excellent for use by physicians in their offices and by diagnostic laboratories, it is not the type of test that is readily adaptable for use by the ordinary person because of his adverse reaction to handling fecal matter and because of his lack of skill in interpreting the results. As stated above, the Pagano test requires that a specimen of fecal matter be obtained. Normally, a specimen is obtained by procuring a sample on the end of a spatula or a wooden depressor, which is then used to smear the specimen on the paper in the Pagano test slide. Once the sample is obtained and the test procedure completed, both the test slide and the spatula or depressor must be disposed of. Disposal of the used materials can and does present a physical problem to, if not an adverse psychological reaction from, the ordinary person. Thus, the ordinary person is not likely to use the Pagano test because of its uncleanly nature (at least apparently so to the ordinary person) and because of the disposal problems associated with the used test slide and spatula or depressor. Additionally, the ordinary person does not have the skill required to analyze, and thus form accurate conclusions from, the test results.

As an alternative, the ordinary person can initiate the Pagano test in his home and then forward the test slide to his physician or a laboratory for addition of the developing agent and analysis of the test. This procedure, however, requires cold storage of the test slide and specimen if there is a significant time lapse before the test can be completed. Certainly, the ordinary person does not wish to store a fecal specimen in his household refrigerator, normally the only cold storage available to him, until he can present the specimen to his physician or an appropriate laboratory. Thus, the general public is not likely to follow or comply with this alternative.

Another test for occult blood is suggested by D. E. Fonner in U.S. Patent No. 2,838,377. The Fonner test, as disclosed, can be effected in a toilet bowl containing fecal matter. The basic test reagents employed by Fonner are o-tolidine and benzidine. These reagents in the presence of blood and other reactants produce a dye visible to the naked eye. Although the Fonner test appears to be a solution to the problem of finding a viable home test for occult blood, it has not met with success for two reasons. First, the above-listed reagents are in themselves known to cause cancer and thus are not suitable for general public distribution. Additionally, the Fonner reagents have a relatively high rate of providing false indications of the presence of occult blood.

Thus, to date, the use of the Pagano test, the Fonner test, and other similar tests has been limited primarily to physicians and diagnostic laboratories. Although this limitation might not at first glance present a significant problem, it does limit the early detection of digestive tract cancers, primarily because patients will not see a physician until other symptoms of digestive tract cancers, such as gross bleeding, manifest themselves. Thus, early detection of cancer of the digestive tract still does not occur with the great majority of patients who contract the disease.

Until the advent of the present invention, the most viable method for testing for occult blood in the home is that disclosed by W. G. Friend in U.S. Patent No. 4,175,932, assigned to Hematec Corporation of Bellevue, Washington. The Friend test again uses the reliable and time-proven test reagent guaiac. In accordance with the preferred embodiment disclosed by Friend, guaiac is impregnated on an absorbent substrate such as an absorbent laboratory filter paper. A developing solution, comprising an alcohol and a peroxide, is applied to the guaiac-containing absorbent substrate. The activated test substrate is then deposited in a toilet bowl, for example, containing feces. If occult blood is present, the guaiac is oxidized to a blue dye that is visible against the absorbent substrate.

While the Friend test overcomes some of the drawbacks of the Pagano test and Fonner test, the Friend test still has its disadvantages. First the alcohol-guaiac solution is highly flammable, presenting a potential hazard to the user in the bathroom, a common smoking area. Secondly, the addition of the solution to the test substrate in both the Pagano and Friend tests will almost always leave a brown or blue-green ring on the substrate unless the solution is evenly distributed over the entire substrate. This ring can easily be misinterpreted by the inexperienced person as a positive test result. Thirdly, the Friend test is a two-step test, requiring the user to not only deposit the substrate in the toilet bowl, but also to first apply the alcohol-guaiac solution to the substrate. As a consequence, it is desirable to eliminate these problems and at the same time provide a one-step test.

British Patent No. 1486088 granted to Lachema N. P. discloses a diagnostic test strip for the detection and semiquantitative estimation of hemoglobin in a biological material, comprising a bibulous carrier containing a chromogen and an organic hydroperoxide in the form of a salt formed with an amine, aminoalcohol or urea. It had previously been necessary for the chromogen and the organic hydroperoxide to be perfectly separated (page 1, lines 81 to 83, of GB—1486088) to avoid unintentional oxidation of the chromogen even in the absence of hemoglobin. However, GB—1486088 discloses the possibility of incorporating both the chromogen and the organic hydroperoxide into the bibulous carrier, and this is made feasible by having the hydroperoxide, not in its free form, but in the form of a salt with an amine, aminoalcohol or urea, so as temporarily to deactivate the oxidising effect of the organic hydroperoxide. In use, in the presence of water, the hydroperoxide becomes free to carry out its oxidising function on the chromogen, provided that hemoglobin is also present to provide the necessary catalytic effect.

GB—1486088 also mentions the optical presence of a solid natural or synthetic film-forming polymer (page 2, lines 96 and 97) provided, inter alia, in order to protect the test area of the diagnostic strip against the influence of air and air humidity (page 3, lines 44 to 46), to prevent moisture causing the hydroperoxide in salt form to become free to oxidise the chromogen, when the strip is stored in a damp atmosphere.

According to the present invention, there is provided a diagnostic aid for use in determining the presence of hemoglobin in an aqueous environment, the diagnostic aid comprising: a composition comprising an absorbent cellulosic material having absorbed guaiac therein, and an oxidising agent, the oxidising agent being capable of oxidising the guaiac to a blue dye in the presence of water and hemoglobin; characterised:— in that the guaiac is carried in a polymer matrix, the polymer being soluble in water; in that the oxidising agent as such in dry form is in direct contact with the composition, the oxidising agent in the absence of water being substantially non-reactive with the guaiac in the composition; and in that the diagnostic aid is substantially dry and can be utilised in a single step to ascertain the presence of hemoglobin in an aqueous environment.

The present invention achieves the desired result by providing a diagnostic aid employing the guaiac reaction that for use requires only the single manipulative step of depositing a single reagent package in an aqueous medium to be tested for the presence of occult blood. Once the package is in the aqueous medium, it is observed for the characteristic blue dye reaction to determine whether occult blood is present in the aqueous medium. All of the necessary components for causing guaiac to react in an aqueous medium are present in the diagnostic aid in a dry form that does not require the addition of a liquid developer by the user, as has been required by all prior tests employing guaiac since the guaiac reaction was discovered over one hundred years ago.

In the diagnostic aid of the present invention, the guaiac in a polymer matrix is absorbed into the absorbent cellulosic carrier material. The polymer must be soluble in water and is also preferably soluble in a solvent for guaiac. In a preferred form, the absorbent cellulosic carrier material comprises a sheet of absorbent cellulosic material containing the guaiac in a polymer matrix, onto which sheet the oxidising agent in a dry particulate form has been spread.

The diagnostic aid in accordance with the present invention is inexpensive, clean noncarcinogenic, and is otherwise safe to handle. The aid has a long shelf life and is very simple to use. Since a positive reaction by the aid yields a bright blue dye that is highly visible against the background of the carrier and/or the oxidising agent, the test is easily interpreted with little likelihood of error. Moreover, the user of the aid need only to perform a single manipulative step before observing to see whether the guaiac reaction with hemoglobin has occurred.

The diagnostic aid manufactured in accordance with the present invention places all of the components necessary to yield a positive guaiac reaction in the presence of hemoglobin (one that produces a blue dye) into a single reagent package containing only dry ingredients. By dry, it is meant substantially dry in appearance and to the touch. This definition includes, as will be understood when the entire specification has been read, a liquid absorbed onto an absorbent substrate. To use the diagnostic aid, it need only be deposited in an aqueous medium containing the material to be tested for the presence of occult blood, for example, a toilet bowl containing feces.

The present invention provides a single reagent package comprising (a) an absorbent carrier into which has been absorbed guaiac distributed through a polymer matrix, that is in admixture with a polymer, and (b) a dry oxidising agent capable of producing the required oxidising compound upon contact with the aqueous medium in which the test is to be conducted. It is preferred that the carrier be a coherent, absorbent substrate serving both as the absorbent carrier and as a substrate onto which the oxidising agent can readily be applied. It is preferred that the substrate be white or some other neutral colour that provides a background against which the blue dye produced by the guaiac reaction can readily be observed by the naked eye. The absorbent substrate can take a variety of forms although an absorbent cellulosic mat or sheet is especially efficacious. Preferred cellulosic sheet materials include commercially available filter and chromatography paper such as Whatman No. 1 chromatography paper available from the Whatman Paper Division of Clifton, New Jersey 07014. Although, an absorbent sheet is presently preferred as a carrier, a ball or mass of absorbent material can be affixed to one end of a swab or stick and can function equally well as a substrate for the guaiac reagent system.

The absorbent carrier serves as a storage medium and reaction site to hold the guaiac in a form in which it is readily oxidisable to its characteristic blue dye. Guaiac alone absorbed onto a paper substrate will too readily react with available oxygen and prematurely turn blue or bluish-green, especially if exposed to sunlight. It has been found, however, when the guaiac is dispersed in a water soluble polymer matrix and then absorbed onto a paper substrate, that the guaiac is presented in a manner in which it is readily oxidisable to a blue dye in the presence of hemoglobin. The polymer, however, prevents premature auto oxidation and consequently gives the diagnostic aid a long shelf life, a necessary characteristic in a commercially viable product.

The guaiac is dispersed in the polymer and absorbed onto the absorbent carrier so that it is present in a polymer matrix by preferably first dissolving guaiac in a solvent in which both the guaiac and the polymer are soluble. The polymer is introduced into the guaiac solvent system to form a solution. This solution is then applied to the absorbent carrier, after which the solvent is evaporated, leaving the absorbent carrier containing the guaiac in the polymer matrix. Both water-miscible and water-immiscible solvents are effective for dissolving the guaiac and polymer for introduction into the absorbent carrier. Suitable water-miscible solvents include alkanols and alkenols having from 1 to 6 carbon atoms, and particularly the lower aliphatic alcohols. The most preferred alcohols include methanol, ethanol, isopropyl alcohol and mixtures thereof. Water-immiscible solvents that may be employed include chloroform and benzene. It is preferred, of course, to employ a solvent that has a relatively high vapor pressure so that the solvent can be readily evaporated from the absorbent carrier.

Suitable polymers that can be employed in accordance with this embodiment of the invention must be both water soluble and soluble in the solvent for guaiac. Such polymers include polyethyleneglycol and polyvinylpyrollidone. Polyethyleneglycol can be employed in molecular weights ranging from about 200 to 20,000. Similarly, polyvinylpyrollidone can be utilized in molecular weights ranging from 10,000 to 360,000. Both of these polymers are readily soluble in the lower aliphatic alcohols, the preferred guaiac solvents. If desired, of course, mixtures of these two polymers or of either of these polymers with other polymers that are readily soluble in guaiac solvents can be employed.

One of the primary objectives of the present invention is to produce a dry diagnostic aid that is contained in a single reagent package. Therefore, since the guaiac and polymer absorbed onto the carrier is dry, it is incumbent that the oxidising agent employed in the system also be in dry form. The oxidising agent is preferably of the type that will yield hydrogen peroxide in the presence of water. The hydrogen peroxide, of course, is the oxidant that promotes the reaction between guaiac and hemoglobin to produce the blue reaction product. The oxidising agent must be capable of being combined with the guaiac and polymer containing carrier to form a single reagent package that will readily react in an aqueous system in the presence of hemoglobin. Additionally, the oxidising agent must be relatively inert with respect to the guaiac in the absence of water. Yet, the oxidising agent must readily yield its hydrogen peroxide in the presence of water so that the guaiac reaction will take place in a relatively short time after the diagnostic aid has been inserted into the aqueous reaction medium.

Oxidising agents that will produce hydrogen peroxide in the presence of water and that are dry, stable particulate compositions include monosulfate compounds, peroxide compounds such as barium, strontium and magnesium peroxide, benzoyl peroxide, and potassium perborate.

The presently preferred oxidising agent is potassium monopersulfate, a white, granular, free-flowing powder. This monopersulphate compound is commercially available as a triple salt comprised of two mols of potassium monopersulfate, one mol of potassium hydrogen sulfate, and one mol of potassium sulfate under the trade name Oxone® from E. I. DuPont de Nemours & Company, inc of Wilmington, Delaware. These dry particulate oxidising agents can be applied to the guaiac and polymer containing carrier such as a sheet of absorbent paper by dusting small amounts onto one or both surfaces of the paper carrier.

It has also been found that liquid oxidising agents can be absorbed onto a suitable absorbent substrate, for example, a silica gel. Additionally, some of the soluble peroxide compounds and relatives thereof can also be dissolved and then absorbed onto a similar substrate. After the liquid and dissolved oxidising agent are absorbed onto a suitable substrate, that oxidising agent is then contacted with a guaiac-containing carrier to form a single composition. For example, hydrogen peroxide can be absorbed onto commercially available silica gels such as Syloid®, a trademark of the Davison Chemical Divison of W. R. Grace & Company, Baltimore, Maryland. Additionally, other liquid peroxide compounds such as cumene hydroperoxide and tertiary butyl hydroperoxide can be dissolved in isopropyl alcohol or adsorbed directly onto a silica gel carrier. Although these latter oxidising agents are effective to produce a noticeable and accurate guaiac reaction, none functions as well as the dry monopersulphate compound identified above.

In practice, the preferred embodiment of the present invention can be produced by dissolving guaiac in a solvent therefor. It is preferred that the guaiac be present in, for example, isopropyl alcohol in the range of from 1 to 5 percent by weight based on the total weight of the solution, although more guaiac can be utilised if desired. It is most preferred, however, that from 1 to 3 percent by weight of guaiac be employed in the solvent. At present, the most preferred composition constitutes about 3 percent by guaiac weight in isopropyl alcohol. This guaiac solution is then combined with the polymer that is also soluble in the alcohol. It has been found that the guaiac reaction will occur if a polymer such as polyethyleneglycol or polyvinylpyrollidone is combined with the guaiac solution ranging from 1 percent to 60 percent by volume based on the total solution. However, the best and most economical results are obtained when the polymer is combined with the guaiac solution in an amount ranging from about 5 to about 10 percent by volume.

Once the polymer and guaiac have been dissolved in a mutual solvent, the resulting solution is applied to an absorbent substrate such as Whatman No. 1 chromatography paper. The combined solution of guaiac and polymer is placed on a square of absorbent chromatography paper by applying about .1 to .25 milliliters (5 to 10 drops) to a 5.08cm × 5.08cm (2 inch by 2 inch) paper. The paper is then allowed to air dry, although forced drying at relatively low temperatures can be employed. Once the solvent has evaporated, the guaiac distributed through a polymer matrix remains in the absorbent paper. Although not necessary, it is preferred that the paper with the guaiac and polymer absorbed thereon be allowed to completely dry, for example, by allowing it to stand at room temperature for on the order of from 12 to 24 hours before an oxidising agent is applied.

The dry particulate oxidising agent agents can be applied to the substrate by dusting it on, brushing it on, or by other suitable methods for applying a powder to an absorbent paper substrate. Usually no more than the order of 25 to 50 milligrams of potassium monopersulfate need be applied to 5.08 cm by 5.08 cm (two inch by two inch) square to produce an activated substrate. It is preferred that an excess amount of the oxidising agent be distributed over the substrate surface relative to the amount of guaiac present in substrate. Preferably, the monopersulfate oxidising agent is employed in an amount ranging from about 5 to 25 parts by weight per part by weight of guaiac present in the substrate or carrier. Similarly, if a liquid peroxide absorbed onto a silica gel carrier is employed, an amount of the peroxide compound that is the weight equivalent of the monopersulfate compound can be utilized. Thus, for example, 25 to 50 milligrams of cumene hydroperoxide or tertiary butyl hydroperoxide absorbed onto a sufficient amount of silica gel to produce a dry powder can be substituted for the preferred monopersulfate.

At present, the most preferred embodiment of the invention is produced by combining 2 percent by weight of guaiac with isopropyl alcohol. The percentage of guaiac is based on the total weight of the solution. Polyethyleneglycol having a molecular weight of about 400 is then utilized to dilute the solution to about 10 percent by volume polyethyleneglycol and 90 percent by volume alcohol-guaiac solution. About 8 drops of this solution is then applied to a 5.08 cm by 5.08 cm (two inch by two inch) square of chromatography paper. The alcohol is then allowed to evaporate. Subsequently about 50 milligrams of a triple salt of potassium monopersulfate identified as Oxone® above is dusted over the 5.08 cm by 5.08 cm (two inch by two inch) square. The activated test substrate prepared in accordance with the first embodiment of the invention is then available and ready for detecting the presence of hemoglobin in an aqueous environment such as a toilet bowl containing feces.

As will be readily recognized by one of ordinary skill, the present invention as exemplified by the foregoing embodiment, represents a significant advance over prior art diagnostic aids employing the guaiac reaction. All that need be done with the single composition system, which includes the guaiac and polymer containing cellulosic carrier and oxidising agent, is to place the composition in an aqueous medium, such as a toilet bowl, the contents of which are to be tested for occult blood, and to observe the composition for the characteristic color change. The diagnostic aid and method for determining the presence of occult blood in accordance with the present invention do not necessarily require the handling of feces samples or the application of the same to a paper substrate. Neither does the present invention require the handling or application of a liquid developing solution to a substrate or carrier as all prior art requires. Instead, in accordance with the present invention, a single, dry package containing all of the required test reactants is placed in contact with the aqueous medium and thereafter observed for the blue dye that results from the guaiac oxidation reaction. Additionally, a positive reaction with the diagnostic aid prepared in accordance with the present invention yields a bright, vivid blue dye that is very easy to interpret, even by the inexperienced home user.

Although the present invention has been described in terms of a preferred embodiment and various alternatives thereto, one of ordinary skill after reading the foregoing specification will be able to effect various changes, substitutions of equivalents, and other alterations without departing from the broad concepts disclosed herein. For example, a small amount of hemoglobin can be incorporated into the diagnostic aid as a comparison control to assist the ordinary person in correctly interpreting a positive test result. The hemoglobin can be impregnated into a designated location on the filter paper in which reactant compositions are packaged. The user of the aid, of course, would be instructed of the location of the control so that the blue dye produced by the presence of hemoglobin at the designated location would not be mistaken for a positive test, but rather would be used only as an interpretation aid.

**Claims**

1. A diagnostic aid for use in determining the presence of hemoglobin in an aqueous environment, the diagnostic aid comprising: a composition comprising an absorbent cellulosic material having absorbed guaiac therein, and an oxidising agent, the oxidising agent being capable of oxidising the guaiac to a blue dye in the presence of water and hemoglobin; characterised:— in that the guaiac is carried in a polymer matrix, the polymer being soluble in water; in that the oxidising agent as such in dry form is in direct contact with the composition, the oxidising agent in the absence of water being substantially non-reactive with the guaiac in the composition; and in that the diagnostic aid is substantially dry and can be utilised in a single step to ascertain the presence of hemoglobin in an aqueous environment.

2. A diagnostic aid according to Claim 1, further characterised in that the cellulosic material is in the form of a sheet.

3. A diagnostic aid according to Claim 2, further characterised in that the oxidising agent is present as a layer on the sheet of cellulosic material.

4. A diagnostic aid according to Claim 2 or 3, further characterised in that the polymer is selected from polyethylene glycol, polyvinylpyrollidone, and mixtures thereof, and is preferably a polyethylene glycol having a molecular weight in the range from 200 to 20,000 or a polyvinylpyrollidone having a molecular weight in the range from 10,000 to 360,000.

5. A diagnostic aid according to any preceding claim, further characterised in that the oxidising agent is selected from a monopersulphate compound, cumene hydroperoxide, tertiary butyl hydroperoxide, and mixtures thereof, the oxidising agent preferably being a monopersulfate compound, and more preferably being potassium monopersulfate or a mixture of potassium monopersulfate, potassium hydrogen sulphate, and potassium sulphate.

6. A method of forming a diagnostic aid according to any preceding claim, characterised in that the polymer matrix carrying the guaiac is formed by dissolving the polymer and the guaiac in a mutual solvent, to provide a solution, applying the solution to the cellulosic material and evaporating substantially all of the solvent, the solvent preferably being selected from a lower aliphatic alcohol, chloroform, benzene, and mixtures thereof, the lower aliphatic alcohol preferably being an alkanol or alkenol having from one to six carbon atoms, more preferably methanol, isopropanol or a mixture thereof; and in that a substantially dry oxidising agent is then applied to the cellulosic material in which is already absorbed the guaiac carried in the polymer matrix.

7. A method according to Claim 6, characterised in that the guaiac is present in the solvent in an amount in the range from 1 to 5% by weight, preferably from 1 to 3% by weight, and in that the polymer is present in the solvent in an amount in the range from 1 to 60% by volume.

8. A method for determining the presence of hemoglobin in an aqueous environment, the method comprising: placing in contact with the aqueous environment, a diagnostic aid according to any one of Claims 1 to 5; and observing whether a portion of the diagnostic aid is dyed blue.

**Patentansprüche**

1. Diagnosemittel zur Verwendung bei der Bestimmung der Anwesenheit von Hämoglobin in einer wäßrigen Umgebung, enthaltend ein Mittel aus einem absorbierenden Cellulosematerial mit darin absorbiertem Guajac und ein Oxidationsmittel, das das Guajac in Gegenwart von Wasser und Hämoglobin zu einem blauen Farbstoff zu oxidieren vermag, dadurch gekennzeichnet, daß sich das Guajac in einer Matrix eines wasserlöslichen Polymerisats befindet, daß das Oxidationsmittel als solches in trockener Form in direkter Berührung mit dem Mittel steht, daß das Oxidationsmittel in Abwesenheit von Wasser mit dem in dem Mittel enthaltenen Guajac praktisch nicht reagiert und daß das Diagnosemittel praktisch trocken ist und in einem einzigen Schritt zur Sicherstellung der Anwesenheit von Hämoglobin in einer wäßrigen Umgebung verwendet werden kann.

2. Diagnosemittel nach Anspruch 1, dadurch gekennzeichnet, daß das Cellulosematerial in Lagenform vorliegt.

3. Diagnosemittel nach Anspruch 2, dadurch gekennzeichnet, daß das Oxidationsmittel als Schicht auf der Lage aus dem Cellulosematerial vorliegt.

4. Diagnosemittel nach Ansprüchen 2 oder 3, dadurch gekennzeichnet, daß das Polymerisat aus Polyethylenglykol und/oder Polyvinylpyrrolidon ausgewählt ist und vorzugsweise aus einem Polyethylenglykol eines Molekulargewichts im Bereich von 200—20, 000 oder einem Polyvinylpyrrollidon eines Molekulargewichts im Bereich von 10 000—360 000 besteht.

5. Diagnosemittel nach einem der vorhergehenden Ansprüche, dadurch gekennzeichnet, daß das Oxidationsmittel aus einer Monopersulfatverbindung, Cumolhydroperoxid, tert.-Butylhydroperoxid oder einer Mischung derselben ausgewählt ist und zweckmäßigerweise weise aus einer Monopersulfatverbindung, vorzugsweise aus Kaliummonopersulfat oder einer Mischung aus Kaliummonopersulfat, Kaliumhydrogensulfat und Kaliumsulfat besteht.

6. Verfahren zur Herstellung eines Diagnosemittels nach einem der vorhergehenden Ansprüche, dadurch gekennzeichnet, daß man die das Guajac tragende Polymerisatmatrix durch Auflo-4sen des Polymerisats und des Guajacs in einem gemeinsamen Lösungsmittel, Applikation der erhaltenen Lösung auf das Cellulosematerial und Verdampfen praktisch des gesamten Lösungsmittels herstellt, wobei das Lösungsmittel vorzugsweise aus einem kurzkettigen aliphatischen Alkohol, zweckmäßigerweise einem Alkanol oder Alkenol mit 1 bis 6 Kohlenstoffatom(en), vorzugsweise Methanol und/oder Isopropanol, Chloroform, Benzol oder Mischungen derselben ausgewählt ist, und daß man danach auf das Cellulosematerial, in welchem bereits das in der Polymerisatmatrix befindliche Guajac absorbiert ist, ein praktisch trockenes Oxidationsmittel appliziert.

7. Verfahren nach Anspruch 6, dadurch gekennzeichnet, daß das Guajac in dem Lösungsmittel in einer Menge im Bereich von 1 bis 5, vorzugsweise von 1 bis 3 Gew.-%, und das Polymerisat in dem Lösungsmittel in einer Menge im Bereich von 1 bis 60 Vol.-% enthalten ist.

8. Verfahren zur Ermittlung der Anwesenheit von Hämoglobin in einer wäßrigen Umgebung, dadurch gekennzeichnet, daß man mit der wäßrigen Umgebung ein Diagnosemittel nach einem der Ansprüche 1 bis 5 in Berührung bringt und beobachtet, ob sich ein Teil des Diagnosemittels blau färbt.

**Revendications**

1. Un agent auxiliaire de diagnostic utilisable dans la détermination de la présence d'hémoglobine dans un milieu aqueux, cet agent auxiliaire de diagnostic comprenant: une composition qui contient un matériau cellulose absorbant dans lequel est absorbé du gaiac et un agent d'oxydation, l'agent d'oxydation étant capable d'oxyder le gaiac en lui conférant une coloration bleue en la présence d'eau et hémoglobine, caractérisé en ce que le gaiac est fixé sur une matrice polymère, le polymère étant soluble dans l'eau, et ence que l'agent d'oxydation, tel quel sous forme sèche, est directement au contact de la composition, l'agent d'oxydation étant en l'absence d'eau sensiblement non réactif avec le gaiac de la composition et en ce que l'agent auxiliaire de diagnostic est sensiblement sec et permet en une seule étape de mettre en évidence la présence d'hémoglobine dans un environnement aqueux.

2. Un agent auxiliaire de diagnostic selon la revendication 1, caractérisé en ce que le matériau cellulosique se présente sous la forme d'une feuille.

3. Un agent auxiliaire de diagnostic selon la revendication 2, caractérisé en ce que l'agent d'oxydation est disposé sous la forme d'une couche sur la feuille de matériau cellulosique.

4. Un agent auxiliaire de diagnostic selon la revendication 2 ou 3, caractérisé en ce que le polymère est choisi parmi le polyéthylèneglycol, le polyvinylpyrrolidone et leurs mélanges et qu'il s'agit de préférence d'un polyéthylèneglycol dont le poids moléculaire est compris entre 200 et 20 000 ou d'un polyvinylpyrrolidone dont le poids moléculaire est compris entre 10 000 et 360 000.

5. Un agent auxiliaire de diagnostic selon l'une quelconque des revendications précédentes, caractérisé en ce que l'agent oxydant est choisi dans le groupe formé par les composés à base de monopersulfate, l'hydroperoxyde de cumène, l'hydroperoxide de tertiobutyle et leurs mélanges, l'agent oxydant étant de préférence un composé à base de monopersulfate et étant plus particulièrement le monopersulfate de potassium ou un mélange de monopersulfate de potassium, de sulfate acide de potassium et de potassium.

6. Une méthode de réalisation d'un agent auxiliaire de diagnostic selon l'une des revendications précédentes, caractérisé en ce que la matrice polymère qui supporte le gaiac est formée par dissolution du polymère et du gaiac dans un solvant mutuel conduisant à une solution, en ce que l'on applique la solution au matériau cellulosique et en ce que l'on évapore sensiblement tout le solvant, ce solvant étant de préférence sélectionné parmi les alcools aliphatiques inférieurs, le chloroforme, le benzène et leurs mélanges, l'alcool aliphatique inférieur étant de préférence un alcanol un alcénol comportant de 1 à 6 atomes de carbone, plus particulièrement le méthanol, l'isopropanol ou leurs mélanges, et en ce que l'on applique alors un agent oxydant sensiblement sec au matériau cellulosique dans lequel est déjà absorbé le gaiac fixé à la matrice polymère.

7. Une méthode selon la revendication 6, caractérisé en ce que le gaiac est présent dans le solvant en une quantité comprise entre 1 et 5% en poids, de préférence entre 1 et 3% en poids, et en ce que le polymère est présent dans le solvant en une quantité comprise entre 1 et 60% en volume.

8. Une méthode de détermination de la presence d'hémoglobine dans un environnement aqueux, cette méthode consistant à placer au contact de l'environnement aqueux un agent auxiliaire de diagnostic selon une quelconque des revendications 1 à 5 et à observer si une portion de l'agent auxiliaire de diagnostic est colorée en bleu.